(19) Europäisches Patentamt
European Patent Office
Office européen des brevets

(11) Publication number: 0 418 007 A2

(12) # EUROPEAN PATENT APPLICATION

(21) Application number: 90309864.8

(22) Date of filing: 10.09.90

(51) Int. Cl.5: C07D 239/54, A61K 31/505

(30) Priority: 11.09.89 GB 8920457

(43) Date of publication of application: 20.03.91 Bulletin 91/12

(84) Designated Contracting States: AT BE CH DE DK ES FR GB GR IT LI LU NL SE

(71) Applicant: GLAXO GROUP LIMITED
Clarges House 6-12 Clarges Street
London W1Y 8DH(GB)

(72) Inventor: Myers, Peter Leslie
Glaxo Group Research Limited, Berkley Avenue
Greenford, Middlesex UB6 0HE(GB)

(74) Representative: Brewer, Christopher Laurence et al
Glaxo Holdings p.l.c. 63 Graham Street
London N1 8JZ(GB)

(54) **Cyclopentane derivatives.**

(57) The present invention provides the compound [1R-(1β,3α,4β)]-5- [(E)-2-bromovinyl]-1-[3-hydroxy-4-(hydroxymethyl)-1-cyclopentyl- 2,4(1H,3H)-pyrimidinedione and salts and solvates and pharmaceutically acceptable derivatives thereof, and describes processes for their preparation, pharmaceutical compositions containing them and their use in the treatment of viral diseases, especially those caused by the Herpetoviridae.

EP 0 418 007 A2

## CYCLOPENTANE DERIVATIVES

This invention relates to a chiral carbocyclic nucleoside having activity against Herpetoviridae, to processes for its preparation, to pharmaceutical formulations containing it and to its use in medicine.

In European Patent No. 0104066 we describe antiviral carbocyclic nucleosides having the general formula (I)

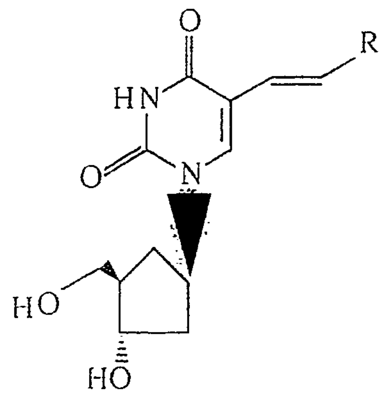

(I)

wherein R represents a chlorine, bromine or iodine atom and the halovinyl group is in the E-configuration, and physiologically acceptable salts thereof with bases.

We have now found that one enantiomer of the racemic bromovinyl compound of Example 1 in European Patent No. 0104066 has excellent activity against Herpetoviridae, especially against strains of both herpes simplex virus type 1 (HSV-1) and varicella-zoster virus (VZV) whilst having a low level of cytotoxicity.

Thus, according to a first aspect of the present invention, there is provided a compound [1R-(1$\beta$,3$\alpha$,4$\beta$)-]-5-[(E)-2-bromovinyl]-1-[3-hydroxy-4-(hydroxymethyl)-1-cyclopentyl-2,4(1H,3H)-pyrimidinedione (hereinafter referred to as 'Compound A') and salts, solvates and pharmaceutically acceptable derivatives thereof.

It will be appreciated that, for pharmaceutical use, the salts referred to above will be the physiologically acceptable salts with bases, but other salts may find use, for example in the preparation of Compound A and the physiologically acceptable salts thereof with bases.

Suitable physiologically acceptable salts of Compound A include inorganic base salts such as alkali metal salts (for example sodium or potassium salts).

By "a pharmaceutically acceptable derivative" is meant any pharmaceutically acceptable ester, ether or salt of such ester or ether of Compound A or any other compound which, upon administration to a mammal, is capable of transformation (directly or undirectly) into Compound A or an antivirally active metabolite or residue thereof.

Preferred esters of Compound A include carboxylic acid esters in which the atom or group attached to the carbonyl moiety of the ester grouping is selected from hydrogen, straight or branched chain alkyl (e.g. methyl, ethyl, n-propyl, t-butyl, n-butyl), alkoxyalkyl (e.g. methoxymethyl), aralkyl (e.g. benzyl), aryloxyalkyl (e.g. phenoxymethyl), aryl (e.g. phenyl optionally substituted by halogen, $C_{1-4}$alkyl or $C_{1-4}$alkoxy); sulphonate esters such as alkyl- or aralkylsulphonyl (e.g. methanesulphonyl); amino acid esters (e.g. L-valyl or L-isoleucyl) and mono-, di- or tri-phosphate esters.

Preferred ethers of Compound A include straight or branched chain $C_{1-4}$alkyl ethers such as isopropyl ethers.

With regard to the above described esters any alkyl moiety present advantageously contains 1 to 18 carbon atoms, particularly 1 to 4 carbon atoms. Any aryl moiety present in such esters advantageously comprises a phenyl group.

References hereinafter to a compound according to the invention includes both the Compound A and salts, solvates and pharmaceutically acceptable derivatives thereof.

It will be understood that all tautomeric forms of Compound A are included within the scope of the invention.

We have found that Compound A is highly potent in vitro against strains of both HSV-1 and VZV and in vivo against strains of HSV-1. In vitro testing was carried out using the standard plaque reduction test whilst in vivo testing was carried out on the mouse according to the method described by Ericson et al . (1985)

Antimicrobial Agents - Chemotherapy 27 , 753-759.

It should be noted that Compound A lacks a glycosidic bond which forms a site for both chemical and biological cleavage. Stability against glycosidic cleavage is, of course, a valuable feature in compounds for *in vivo* use.

In view of its activity profile, Compound A and its physiologically acceptable salts and solvates and pharmaceutically acceptable derivatives thereof recommend themselves for the treatment of a variety of primary and recurrent infections caused by Herpetoviridae, including diseases such as stomatitis, skin eruptions, chicken pox, shingles, encephalitis, eye herpes infections, retinitis, pneumonitis and HSV-1 related genital herpes infections.

The present invention further includes Compound A or a physiologically acceptable salt or solvate thereof or a pharmaceutically acceptable derivatives thereof for use in the treatment or prophylaxis of human viral infections caused by Herpetoviridae, especially those infected caused by HSV-1 or, in particular, VZV.

According to a further aspect of the invention there is provided the use of Compound A or a physiologically acceptable salt or solvate thereof or a pharmaceutically acceptable derivative thereof for the manufacture of a medicament for the treatment or prophylaxis of human viral infections caused by Herpetoviridae, especially those infections caused by HSV-1 or, in particular, VZV.

According to another aspect of the invention there is provided a method of treatment of a human body to oombat Herpetoviridae, especially HSV-1 or, in particular, VZV, which method comprises administering to the body an effective amount of Compound A or physiologically acceptable salt or solvate thereof or a pharmaceutically acceptable derivative thereof.

Compound A and its physiologically acceptable salts and solvates and pharmaceutically acceptable derivatives thereof may be formulated for administration in any convenient way, and the invention therefore also includes within its scope pharmaceutical compositions for use in the therapy or prophylaxis of Herpetoviridae (e.g. HSV-1 or, in particular, VZV) infections, in a human subject comprising Compound A or a physiologically acceptable salt or solvate thereof or a pharmaceutically acceptable derivative thereof together, if desirable, with one or more physiologically acceptable carriers or excipients.

Compounds of the invention may, for example, be formulated for oral, buccal, parenteral, topical or rectal administration.

Tablets and capsules for oral administration may contain conventional excipients such as binding agents, for example syrup, acacia, gelatin, sorbitol, tragacanth, mucilage of starch or polyvinyl pyrrolidone; fillers, for example, lactose, sugar, maize-starch, calcium phosphate or sorbitol; lubricants, for example, magnesium stearate, talc, polyethylene glycol or silica; disintegrants, for example, potato starch or sodium starch glycollate; or wetting agents such as sodium lauryl sulphate. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives (such as suspending agents), for example, sorbitol syrup, methyl cellulose, glucose/sugar syrup, gelatin, hydroxymethyl cellulose, carboxymethyl cellulose, aluminium stearate gel or hydrogenated edible fats; emulsifying agents, for example, lecithin, sorbitan mono-oleate or acacia; non-aqueous vehicles (which may include edible oils), for example almond oil, fractionated coconut oil, oily esters, propylene glycol or ethyl alcohol; or preservatives, for example, methyl or propyl *p* -hydroxybenzoates or sorbic acid. The compound may also be formulated as suppositories, e.g. containing conventional suppository bases such as cocoa butter or other glycerides.

For buccal administration the compositions may take the form of tablets or lozenges formulated in conventional manner.

Compounds of the invention may also be formulated for injection and may be presented in unit dose form, for instance as ampoules, vials, small volume infusions or pre-filled syringes, or in multi-dose containers with an added preservative. The compositions may take such forms as solutions, suspensions, or emulsions in aqueous or non-aqueous vehicles, and may contain formulatory agents such as antioxidants, buffers, antimicrobial agents and/or toxicity adjusting agents. Alternatively, the active ingredient may be in powder form for constitution with a suitable vehicle, e.g. sterile, pyrogen-free water, before use. The dry solid presentation may be prepared by filling a sterile powder aseptically into individual sterile containers or by filling a sterile solution aseptically into each container and freeze-drying.

For topical administration compounds of the invention may be formulated as ointments, creams, lotions, powders, pessaries, sprays, aerosols or drops (e.g. eye or nose drops). Ointments and creams may, for example, be formulated with an aqueous or oily base with the addition of suitable thickening and/or gelling agents. Such bases may thus, for example, include water and/or an oil such as liquid paraffin or a vegetable

oil such as arachis oil or castor oil. Thickening agents which may be used include soft paraffin, aluminium stearate, cetostearyl alcohol, polyethylene glycols, microcrystalline wax and beeswax.

Lotions may be formulated with an aqueous or oily base and will in general also contain one or more emulsifying agents, stabilizing agents, dispersing agents, suspending agents or thickening agents.

Powders for external application may be formed with the aid of any suitable powder base, for example, talc, lactose or starch. Drops may be formulated with an aqueous or non-aqueous base also comprising one or more dispersing agents, solubilising agents or suspending agents.

Aerosol sprays are conveniently delivered from pressurised packs, with the use of a suitable propellant, e.g. dichlorodifluoromethane, trichlorofluoromethane, dichlorotetrafluoroethane, carbon dioxide or other suitable gas.

The pharmaceutical compositions according to the invention may also contain other active ingredients such as antimicrobial agents, or preservatives.

The compositions may contain from 0.1%-99% of the active material. For topical administration, for example, the composition will generally contain from 0.01% to 20%, more preferably 0.5% to 5% of the active material.

For topical administration the daily dosage as employed for adult human treatment will range from 0.1 mg to 1000 mg, preferably 0.5 mg to 10 mg. However, it will be appreciated that extensive skin infections may require the use of higher doses.

For systemic administration the daily dosage as employed for adult human treatment will range from 5 mg to 5000 mg, preferably 50 mg to 2000 mg, which may be administered in 1 to 5 daily doses, for example, depending on the route of administration and the condition of the patient. When the compositions comprise dosage units, each unit will preferably contain 2 mg to 2000 mg of active ingredient, for example 50 mg to 500 mg. For serious infections the compound may be administered by intravenous infusion using, for example 0.01 to 10 mg/kg/hr of the active ingredient.

Compounds of the invention may be administered in combination with one or more further therapeutic agents such as a different antiviral agent.

The invention thus provides, in a further aspect, a combination comprising Compound A or a physiologically acceptable salt or solvate or a pharmaceutically acceptable derivative thereof together with another therapeutically active agent, in particular an antiviral agent.

The combinations referred to above may conveniently be presented for use in the form of a pharmaceutical formulation and thus pharmaceutical formulations comprising a combination as defined above together with a pharmaceutically acceptable carrier thereof comprise a further aspect of the invention.

The individual components of such combinations may be administered either sequentially or simultaneously in separate or combined pharmaceutical formulations.

When a compound of the invention is used in combination with a second therapeutic agent active against the same virus the dose of each compound may differ from that when the compound is used alone. Appropriate doses will be readily appreciated by those skilled in the art.

According to another aspect of the present invention, we provide a process for the preparation of Compound A or a salt, solvate or pharmaceutically acceptable derivative thereof. Thus, one process for the preparation of Compound A or a salt, solvate or pharmaceutically acceptable derivative thereof comprises treating the 1R isomer of a compound of formula (II)

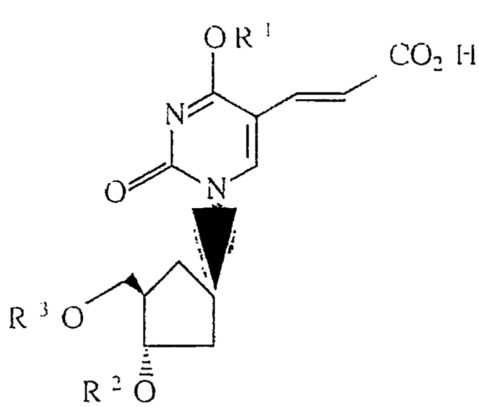

(II)

or a salt thereof (where $R^1$, R2 and R3, which may be the same or different, represent hydrogen atoms or

protecting groups) with a brominating agent followed where necessary by removal of any protecting groups present, with salt formation and conversion to a pharmaceutically acceptable derivative of Compound A as optional subsequent steps.

The reaction is conveniently effected in a medium such as water; an alcohol, e.g. methanol or ethanol; a halogenated hydrocarbon, e.g. chloroform or carbon tetrachloride; or a substituted amide, e.g. N,N-dimethylformamide, and at a temperature in the range of 0° to +100° C, preferably +30° to +70° C.

Suitable reagents for the bromination include an N-bromo amide or imide such as N-bromosuccinimide, N-bromophthalimide, N-bromoacetamide, N-bromocaprolactam or 1,3-dibromo-5,5-dimethyl- hydantoin.

The treatment of 1R isomer of a compound of formula (II) with a brominating agent is conveniently carried out in the presence of a base such as an alkali metal bicarbonate (e.g. potassium bicarbonate).

When the 1R isomer of the compound of formula (II) is used in the form of a salt the salt may be formed with an inorganic or organic base. Suitable salts include alkali metal salts, e.g. sodium or potassium salts; alkaline earth metal salts, e.g. calcium salts; or organic base salts, e.g. triethylamine or pyridine salts.

Where $R^1$, R2 or R3 represents a protecting group, it may be any conventional protecting group, for example, as described in 'Protective Groups in Organic Chemistry', Ed. J. F. W. McOmie (Plenum Press, 1973). Examples of suitable protecting groups are alkyl groups such as methyl, t-butyl or methoxymethyl; aralkyl groups such as benzyl, p-methoxybenzyl, diphenylmethyl or triphenylmethyl; heterocyclic groups such as tetrahydropyranyl; acyl groups such as acetyl and silyl groups such as trialkylsilyl groups, e.g. trimethylsilyl.

The protecting groups may be removed by using conventional techniques to yield Compound A. Thus, for example, an alkyl, aryl, silyl or heterocyclic group may be removed by solvolysis, e.g. hydrolysis under acidic or basic conditions, and an aralkyl group may be cleaved with a boron trihalide e.g. boron trichloride in a solvent such as methylene chloride and at low temperature or by catalytic hydrogenolysis using for example a palladium catalyst such as palladium-on-carbon conveniently in an alcohol solvent (e.g. methanol) and in the presence of a mineral acid such as hydrochloric acid.

The 1R isomer of a compound of formula (II) may be prepared by deprotection of the carboxyl group of a 1R isomer of a compound of formula (III):

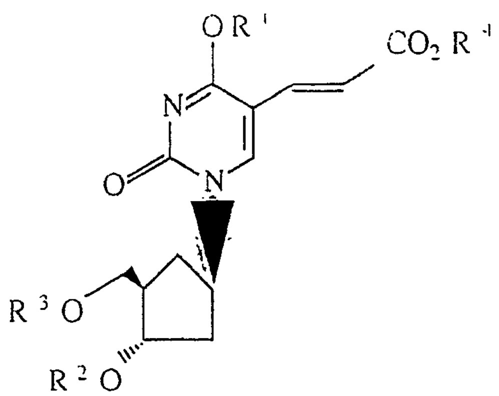

(III)

(where $R^1$, R2 and R3 are as previously defined and $R^4$ represents a carboxyl protecting group).

$R^4$ may be any conventional carboxyl protecting group such as an alkyl group, e.g. methyl or ethyl, or an aralkyl group, e.g. benzyl. The group $R^4$ may be cleaved by conventional means, e.g. by hydrolysis under basic conditions (using for example aqueous sodium hydroxide). The deprotection will generally be effected in the temperature range 0 to +50° C. If desired, removal of the protecting group $R^4$ may also be accompanied by removal of any protecting groups $R^1$, R2 or R3 where present.

The 1R isomer of a compound of formula (III) may be prepared by reaction of a 1R isomer of a compound of formula (IV)

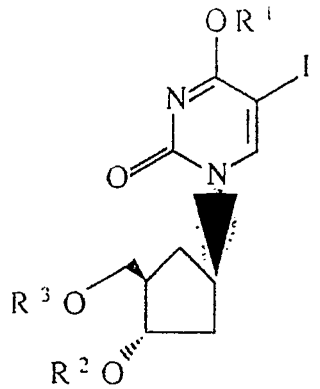

(IV)

(where $R^1$, R2 and R3 are as previously defined) with an ester of acrylic acid:

$CH_2 = CHCO_2R^4$

(where $R^4$ is as previously defined).

The reaction is conveniently carried out in the presence of a transition metal complex such as palladium (II) acetate and a triarylphosphine such as triphenylphosphine in a solvent such as an ether (e.g. dioxan) and at an elevated temperature (e.g. 50° to 100° C). Preferably, an organic base such as an amine (e.g. triethylamine) is also present.

The 1R isomer of a compound of formula (IV) may be prepared by reaction of a 1R isomer of a compound of formula (V)

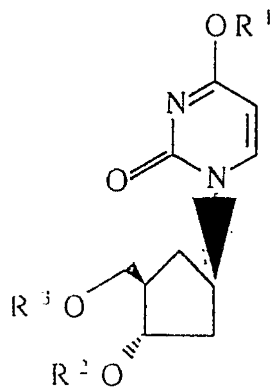

(V)

(where $R^1$, R2 and R3 are as previously defined) with iodine in the presence of nitric acid at an elevated temperature (e.g 50° to 120° C). The reaction is conveniently effected in an ether solvent such as dioxane.

The 1R isomer of a compound of formula (V) may be prepared by reacting the 1S isomer of a compound of formula (VI)

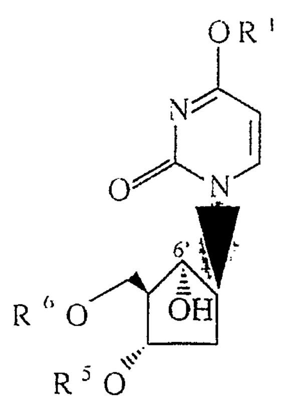

(VI)

(where $R^1$ is as previously defined and $R^5$ and $R^6$ are protecting groups as previously defined for $R^1$, R2

and R3) to convert the 6′-OH group to a leaving group removable by reduction (e.g. by homolytic reduction) and reducing said compound to replace the leaving group by a hydrogen atom, followed if desired by removal of one or more of the protecting groups present.

Conveniently, the 1S isomer of a compound of formula (VI) is reacted with a compound of formula HalC(=S)$OR^7$ (where Hal is a halogen atom, e.g. chlorine, and $R^7$ is $C_{1-6}$alkyl, aryl such as phenyl, heteroaryl such as imidazole or $C_{1-6}$alkylaryl such as p-tolyl), preferably in the presence of a base such as an amine (e.g. 4-dimethylaminopyridine) and in a solvent such as a halogenated hydrocarbon (e.g. dichloromethane) to provide the 1S isomer of a compound of formula (VII)

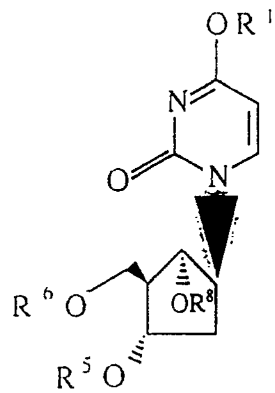

(VII)

(where $OR^8$ represents a group OC(=S)$OR^7$ and $R^1$, $R^5$, $R^6$ and $R^7$ are as previously defined). This compound may thereafter be treated with a reducing agent such as an alkyltin hydride (e.g. tri-n-butyltin hydride) in the presence of a radical initiator such as a peroxide, azobisisobutyronitrile or light and in a suitable solvent such as toluene to provide (if desired after removal of one or more of the protecting groups present) the 1R isomer of a compound of formula (V).

The 1S isomer of a compound of formula (VI) may be prepared by reacting the 1S isomer of a compound of formula (VIII)

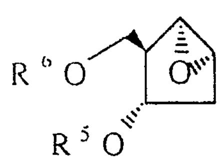

(VIII)

(where $R^5$ and $R^6$ are as previously defined) with a compound of formula (IX)

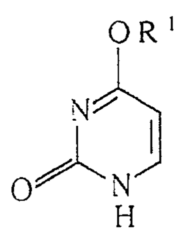

(IX)

(where $R^1$ is as defined previously) in the presence of a suitable base such as an alkali metal hydride (e.g. sodium hydride) and conveniently in a solvent such as dimethylformamide when sodium hydride is the base. The reaction is conveniently carried out at an elevated temperature (e.g. 50°-180°C).

The 1S isomer of a compound of formula (VIII) may be prepared by introduction of a protecting group $R^5$ into a 1S isomer of a compound of formula (X)

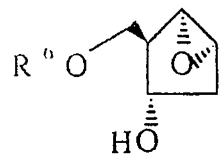

(X)

(where $R^6$ is as previously defined).

Compounds of formula (IX) are either known compounds or may be prepared by methods analogous to those used for the preparation of the known compounds of formula (IX).

Compounds of formula (X) are either known compounds described in J. Chem. Soc. Perkin Trans 1, 1988, 549 or may be prepared by methods analogous to those described therein for the preparation of the known compounds of formula (X).

The optically active isomers of compounds of formulae (II)-(VIII) described above and in the Examples section hereinafter are novel compounds and form a further feature of the present invention.

Base salts of Compound A including physiologically acceptable base salts may be prepared by reacting Compound A with a suitable base (e.g. a hydroxide such as sodium hydroxide) in a suitable solvent (e.g. water).

Physiologically acceptable base salts may also be prepared from other base salts, including other physiologically acceptable base salts, of Compound A using conventional ion exchange methods.

The compound of formula (I) may be converted into a pharmaceutically acceptable derivative thereof by conventional means. Thus, for example, a pharmaceutically acceptable phosphate or other ester may be prepared by reacting a compound of formula (I) with a phosphorylating agent, such as $POCl_3$, or a suitable esterifying agent, such as an acid halide or anhydride, as appropriate.

Solvates (e.g. hydrates) of a compound of formula (I) may be formed during the work-up procedure of one of the aforementioned process steps.

The following Examples illustrate the present invention but should not be construed as a limitation of the invention. All temperatures are in °C.

Intermediate 1

[1S-(1α,2β,3α,5α)]-3-[(4-Methoxyphenyl)methoxy]-2-(phenylmethoxy)-methyl-6-oxabicyclo[3.1.0]hexane

A solution of [1S-(2β,3α,5α)]-2-(phenylmethoxy)methyl-6-oxabicyclo[3.1.0]hexan-3-ol [1] (30.00g) in dry tetrahydrofuran was added to a stirred suspension of petrol-washed sodium hydride (from 5.99g as 60% dispersion in mineral oil) in dry tetrahydrofuran (120ml) under nitrogen. After 80 minutes 4-methoxybenzyl chloride (23.44g) was added followed by tetra-n -butylammonium iodide (503mg) then dry N,N-dimethylformamide (30ml) and the resulting mixture was refluxed under nitrogen for 75 minutes. Methanol (10ml) was added to the cooled mixture which was then evaporated and the residue partitioned between diethyl ether (350ml) and water (350ml). The organic extract was washed with acidified water and these aqueous washes back extracted with diethyl ether (2 × 100ml). The combined organic extracts were washed with water, dried over magnesium sulphate, and evaporated. The residue was subjected to column chromatography on silica (Merck 7734), eluted with petroleum ether-diethyl ether mixtures, to give the title product (44.00g); $^1H$ n.m.r. ($CDCl_3$) in ppm 7.22 (2H), 6.83 (2H), 4.48 (2H), 4.39 (2H), 3.87 (1H), 3.79 (3H), 3.54 (1H), 3.46 (1H), 3.40 (2H), 2.59 (1H).

Intermediate 2

[1S-(1β,2α,3β,4α)]-1-[2-Hydroxy-4-](4-methoxyphenyl)methoxy[-3-(phenylmethoxy)methyl-1-cyclopentyl]-2,4-(1H,3H)-pyrimidinedione

To a stirred suspension of 2,4(1H,3H)-pyrimidinedione (7.90g) in dry N,N-dimethylformamide, under

[1]. Preparation described in J. Chem. Soc. Perkin Trans. 1 1988, 549.

nitrogen, was added sodium hydride (0.47g as 60% dispersion is mineral oil). After 1 hour Intermediate 1 (20.00g) in dry N,N-dimethylformamide (50ml) was added and the resulting mixture heated at ca . 145° for 25 hours. The solvent was evaporated and the residue partitioned between ethyl acetate (300ml) and water (300ml). The organic extract was washed with water and brine, dried over sodium sulphate and evaporated. The residue was subjected to column chromatography on silica (Merck 7734), eluted with ethyl acetate-petroleum ether, then neat ethyl acetate, to give the title product (10.01g); ν max ($CHBr_3$) 3300-3600cm$^{-1}$ (OH, NH), 1650-1750 (uracil); $^1$H n.m.r. ($CDCl_3$) in ppm 7.16 (1H), 5.64 (1H), 4.63 (1H), 4.50 (2H), 4.38 (2H), 4.20 (1H), 3.87 (1H), 3.79 (3H), 3.74 (1H), 3.59 (1H).

## Intermediate 3

[1S-(1β,2α,3β,4α)]-1-[4-((4-Methoxyphenyl)methoxy)-2-((phenoxy-thiocarbonyl)oxy)-3-(phenylmethoxy)-methyl-1-cyclopentyl]-2,4(1H,3H)-pyrimidinedione

To an ice-cooled solution of Intermediate 2 (9.00g) and 4-dimethylaminopyridine (2.34g) in dichloromethane (100ml) was added phenyl chlorothioformate (3.77g). After 30 minutes the reaction was allowed to warm to ambient temperature. After a further 2.5 hours more 4-dimethylaminopyridine (1.20g) was added. After 2 hours the solvent was removed by evaporation and the residue subjected to column chromatography on silica (Merck 7734), eluted with ethyl acetate- petroleum ether mixtures, to give the title product (10.84g); $^1$H n.m.r. ($CDCl_3$) in ppm 7.04 (2H), 6.88 (2H), 5.97 (1H), 5.50 (1H), 5.28 (1H), 4.52 (2H), 4.46 (2H), 4.04 (1H), 3.81 (3H).

## Intermediate 4

[1R-(1β,3α,4β)]-1-[4-((4-Methoxyphenyl)methoxy)-3-(phenylmethoxy)- methyl-1-cyclopentyl]-2,4(1H,3H)-pyrimidinedione

A mixture of Intermediate 3 (9.32g), tributyltin hydride (6.91g) and azoisobutyronitrile (0.49g) in toluene (130ml) was de-gassed with nitrogen then heated at 80°. After 30 minutes the solvent was evaporated and the residue was subjected to column chromatography on silica (Merck 7734), eluted with ethyl acetate-petroleum ether mixtures to give the title product (6.38g); λ max($CHC_3$) 268.8nm (E 269); ν max ($CHBr_3$) 3373cm$^{-1}$ (NH), 1620-1750cm$^{-1}$ (uracil).

## Intermediate 5

[1R-(1β,3α,4β)]-1-[3-Hydroxy-4-(hydroxymethyl)-1-cyclopentyl]-2,4 (1H,3H)-pyrimidinedione

A mixture of Intermediate 4 (5.00g) and 10% palladium-on-carbon (5.00g) in methanol (250ml) and 2 N hydrochloric acid (10ml) was stirred under hydrogen for 30 minutes. Insoluble material was removed by filtration, and solvents were removed by evaporation. The residue was dissolved in methanol (40ml) and the resulting solution poured into diethyl ether (400ml). The solvent was decanted off and the precipitated gum washed with diethyl ether (100ml), and taken into methanol (40ml). The solvent was evaporated to give the title product (2.85g); [α]D +8° ( c 1.7, dichloromethane); $^1$H n.m.r. (DMSO-$d_6$) in ppm 7.69 (1H), 5.58 (1H), 4.95 (1H), 3.97 (1H), 3.43 (2H).

## Intermediate 6

[1R-(1β,3α,4β)]-1-[3-Hydroxy-4-(hydroxymethyl)-1-cyclopentyl]-5-iodo-2,4(1H,3H)-pyrimidinedione

A mixture of Intermediate 5 (1.81g), iodine (4.06g) and 1 N -nitric acid (8ml) in dioxan (80ml) was

heated at 100° for 1 hour. The solvent was evaporated and the residue flushed with ethanol and trichloromethane. Ethanol (20ml) was added to the residue and the resulting solid isolated by filtration to give the title product (1.50g); $^1$H n.m.r. (DMSO-$d_6$) in ppm 8.15 (1H), 4.92 (1H), 3.96 (1H), 3.45 (2H).

Intermediate 7

[1R-(1β,3α,4β)]-5-[(E)-2-Carbomethoxyvinyl]-1-[3-hydroxy-4- (hydroxymethyl)-1-cyclopentyl]-2,4(1H,3H)-pyrimidinedione

A mixture of Intermediate 6 (0.95g) and methyl acrylate (0.70ml) in dioxan (25ml) was added to a solution of palladium (II) acetate (0.22g), triphenylphosphine (0.52g) and triethylamine (0.87ml) in dioxan (65ml) which had been heated at 85° for 20 minutes. The resulting mixture was heated at 85° for 8 hours. Additional palladium (II) acetate (0.05g) and triphenylphosphine (0.10g) was added and heating continued for a further 2 hours. The cooled reaction mixture was filtered and the solvent evaporated. The residue was subjected to column chromatography on silica (Merck 7734), eluted with ethyl acetate-methanol to give the title product (0.47g); $^1$H n.m.r. (DMSO-$d_6$) in ppm 8.32 (1H), 7.46 (1H), 6.90 (1H), 4.99 (1H), 4.75 (1H), 4.26 (1H), 3.98 (1H), 3.68 (3H).

Intermediate 8

[1R-(1β,3α,4β)]-5-[(E)-2-Carboxyvinyl]-1-[3-hydroxy-4-(hydroxy- methyl)-1-cyclopentyl]-2,4(1H,3H)-pyrimidinedione

A solution of Intermediate 7 (0.58g) in 2 N -sodium hydroxide (8ml) was stirred at ambient temperature for 1.5 hours. The reaction was acidified to pH 1 with concentrated hydrochloric acid and the resulting precipitate isolated by filtration to give the title product (0.38g); $^1$H n.m.r. (DMSO-$d_6$) in ppm 8.25 (1H), 7.37 (1H), 6.81 (1H), 4.98 (1H), 3.99 (1H).

Example 1

[1R-(1β,3α,4β)]-5-[(E)-2-Bromovinyl]-1-[3-hydroxy-4-(hydroxymethyl)- 1-cyclopentyl-2,4(1H,3H)-pyrimidinedione

A mixture of Intermediate 8 (100mg) and potassium bicarbonate (100mg) in N,N-dimethylformamide (2ml) was added to a solution of N-bromosuccinimide (61mg) in N,N-dimethylformamide (1ml). The resulting mixture was stirred at 21° for 3 hours, then the solvent removed by evaporation. The residue was subjected to column chromatography on silica (Merck 7734), eluted with ethyl acetate-methanol to give the title product (73mg). A portion crystallised from ethanol had m.p. 183°; [α]D + 9° ( c 1.2, methanol); $^1$H n.m.r. (DMSO-$d_6$) in ppm 7.93 (1H), 7.27 (1H), 6.91 (1H), 4.96 (1H), 4.74 (1H), 4.63 (1H).

Example 2

Pharmaceutical compositions

| (1) Topical creams | |
|---|---|
| | % w/v |
| a) Active ingredient | 0.25 |
| b) Butylene glycol | 15.0 |
| c) Glycerol | 2.5 |
| d) Cetostearyl alcohol | 10.0 |
| e) Self emulsifying monostearin | 1.5 |
| f) Polyoxyethylene (2) olelyl ether | 5.0 |
| g) Beeswax | 3.0 |
| h) Chlorocresol | 0.1 |
| Distilled water to | 100.0 |

Heat the water to 70° and dissolve the chlorocresol (h). Melt (d), (e), (f) and (g) together, heating to 70°. Add the melt to the water with stirring. Disperse (a) in a mixture of (b) and (c) and add the dispersion (warmed to 55°) to the bulk mixture. Cool, with stirring, to 35°.

| (2) Eye Ointment | |
|---|---|
| | % w/v |
| Active ingredient | 3.0 |
| Liquid paraffin | 25.00 |
| White soft paraffin | to 100.0 |

Melt the white soft paraffin by heating to 70°. Disperse the active ingredient in the liquid paraffin, warm the dispersion to 55° and add it with stirring to the molten white soft paraffin. Cool, with stirring, to 35°.

| (3) Eye Drops | |
|---|---|
| | % w/v |
| Active ingredient | 0.2 |
| Benzalkonium chloride | 0.01 |
| Sodium chloride | 0.88 |
| Water for injections | to 100.0 |

Dissolve the active ingredient in the water with warming. Cool; dissolve the benzalkonium chloride and sodium citrate. Make the solution up to volume with water. Sterilise the solution by filtration, collect the filtrate and fill (aseptically) into suitable sterile eye drop containers.

| (4a) Oral Tablet | | |
|---|---|---|
| | mg/Tablet | % w/w |
| Target core weight 300mg | | |
| Active ingredient | 100 | 33.3 |
| Maize Starch | 50 | 16.7 |
| Polyvinyl pyrrolidone | 2 | 0.7 |
| Sodium starch glycolate | 7 | 2.3 |
| Magnesium stearate | 2 | 0.7 |
| Lactose monohydrate q.s. | to 300 | to 100.0 |

Sieve the active ingredient and maize starch through a 40 mesh screen. Blend the maize starch with the active ingredient and lactose monohydrate in a suitable blender. Make an aqueous solution of the polyvinyl pyrrolidone in a 5-10% w/v solution. Add this solution to the mixing powders and mix until granulated. Using suitable equipment pass the granulate through a 12 mesh screen. Dry the granules in an oven or in a fluid bed dryer. Screen the dry granules through a 16 mesh screen, and blend in the sodium starch glycolate and magnesium stearate previously sieved through a 60 mesh screen. Compress on appropriate punches on an automatic tablet machine. The tablets may be covered in a thin polymer coat applied by the usual film coating techniques. A pigment may be included in the film coat.

| (4b) Oral Tablet | | |
|---|---|---|
| | mg/tablet | % w/w |
| Target core weight 300mg | | |
| Active ingredient | 100 | 33.3 |
| Sodium starch glycolate | 6 | 2.0 |
| Magnesium stearate | 2 | 0.7 |
| Microcrystalline cellulose | q.s. to 300 | to 100.0 |

Sieve the active ingredient and microcrystalline cellulose through a 40 mesh screen. Sieve the sodium starch glycolate and magnesium stearate through a 60 mesh screen. Blend the powders together in a suitable blender until homogenous. Compress on appropriate punches on an automatic tablet machine. The tablets may be covered in a thin polymer coat applied by the usual film coating techniques. A pigment may be included in the film coat.

| (5) Oral Capsule | | |
|---|---|---|
| | mg/capsule | % w/w |
| Target fill weight 250mg | | |
| Active ingredient | 100 | 40.0 |
| Magnesium stearate | 2 | 0.8 |
| Sodium starch glycolate | 13 | 5.2 |
| Lactose anhydrous | q.s. to 250 | to 100.0 |

Sieve all the ingredients and mix in a suitable blender. Fill into suitable size hard gelatin capsules using an automatic capsule filling machine.

| (6) Oral suspension | |
|---|---|
| | % w/v |
| Active ingredient | up to 5.0 |
| Sorbitan mono-oleate | 1.0 |
| Sucrose | 60.0 |
| Carboxymethyl cellulose | 3.0 |
| Methyl hydroxybenzoate | 0.15 |
| Propyl hydroxybenzoate | 0.02 |
| Citrate buffer | as required to pH 7.0 |
| Colour (optional) | as required |
| Flavour (optional) | as required |
| Distilled water | to 100.0 |

Dissolve the sucrose and hydroxybenzoates in most of the water with the aid of heat. Cool and disperse

the carboxymethyl cellulose in part of the water with stirring. Mix the syrup and carboxymethyl cellulose gel. Dissolve the sorbitan mono-oleate and buffer in the dispersion, with stirring. Disperse the finely divided active ingredient in the resultant mixture. Add the colour and flavour as required. Check the pH and adjust if necessary. Make the mixture to volume and fill into suitable suspension containers.

| (7) Powder (for external application) | |
|---|---|
| | % w/w |
| Active ingredient | 3.0 |
| Silicon dioxide | 2.0 |
| Maize starch | to 100.0 |

Blend the sieved active ingredient, silicon dioxide and the maize starch in a suitable mechanical blender. Fill the resultant powder blend into suitable powder containers.

| (8) Intravenous Injection | |
|---|---|
| | % w/v |
| Active ingredient | 1.0 |
| Sodium chloride | 0.80 |
| Sodium hydroxide | to pH 10.5 |
| Water for Injections to | 100.0 |

Dissolve the sodium chloride in water. Disperse the active ingredient therein and dissolve by adjusting the pH with sodium hydroxide solution. Make up to volume and sterilise by membrane filtration. Fill the solution aseptically into glass ampoules and seal.

| (9) Freeze-dried Intravenous Injection | |
|---|---|
| | Per Vial |
| Active ingredient | 100mg |
| Sodium hydroxide | to pH 10.5 |

Disperse the active ingredient in water and dissolve by adjusting the pH with sodium hydroxide solution. Make to volume such that a 10mg/ml solution of the active ingredient is produced. Sterilise by membrane filtration and fill 10ml volumes into glass vials. Freeze dry the vial contents. Apply rubber closures and aluminium overseals.

In the above pharmaceutical examples the active ingredient is Compound A.

## Claims

1. [1R-(1β,3α,4β)]-5-[(E)-2-Bromovinyl]-1-[3-hydroxy-4- (hydroxymethyl)-1-cyclopentyl-2,4-(1H,3H)-pyrimidinedione and salts, solvates and pharmaceutically acceptable derivatives thereof.

2. A compound according to Claim 1 and physiologically acceptable salts and solvates and pharmaceutically acceptable derivatives thereof for use in therapy.

3. A compound according to Claim 1 and physiologically acceptable salts and solvates and pharmaceutically acceptable derivatives thereof for use in the treatment or prophylaxis of viral infections in a human subject.

4. Use of a compound according to Claim 1 and physiologically acceptable salts and solvates and

pharmaceutically acceptable derivatives thereof for the manufacture of a medicament for the treatment or prophylaxis of Herpetoviridae infections in a human subject.

5. Pharmaceutical compositions for use in the treatment or prophylaxis of Herpetoviridae infections in a human subject comprising a compound as defined in Claim 1 or a physiologically acceptable salt or solvate thereof or a pharmaceutically acceptable derivative thereof together with one or more physiologically acceptable carriers or excipients.

6. Compositions according to Claim 5 formulated for oral, buccal, parenteral, topical or rectal administration.

7. Compositions according to Claim 5 or Claim 6 in the form of dosage units.

8. A process for the preparation of a compound according to Claim 1 or a salt, solvate or pharmaceutically acceptable derivative thereof which comprises treating the 1R isomer of a compound of formula (II)

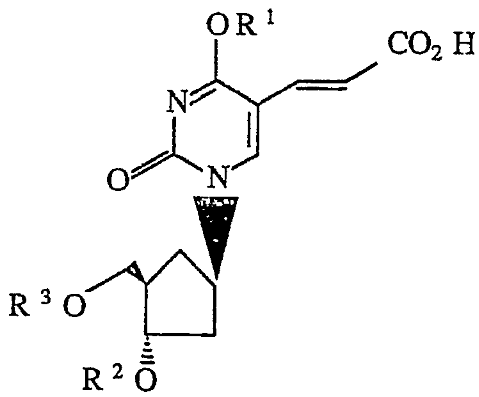

(II)

or a salt thereof (where $R^1$, $R^2$ and $R^3$, which may be the same or different, represent hydrogen atoms or protecting groups) with a brominating agent followed where necessary by removal of any protecting groups present;

with salt formation and conversion to a pharmaceutically acceptable derivative of the compound as defined in Claim 1 as optional subsequent steps.

9. The 1R isomers of the compounds of formulae (II), (III), (IV) and (V).

10. The 1S isomers of the compounds of formula (VI), (VII) and (VIII).

Claims for the following Contracting State: GR

1. A process for the preparation of [1R-(1β,3α,4β)]-5-[(E)-2-bromovinyl]-1-[3-hydroxy-4- (hydroxymethyl)-1-cyclopentyl-2,4-(1H,3H)-pyrimidinedione and salts, solvates and pharmaceutically acceptable derivatives thereof which comprises treating the 1R isomer of a compound of formula (II)

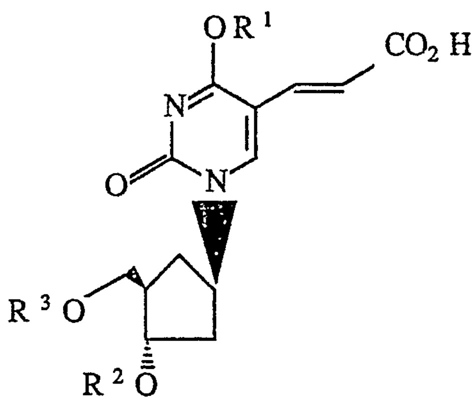

(II)

or a salt thereof (where $R^1$, $R^2$ and $R^3$, which may be the same or different, represent hydrogen atoms or protecting groups) with a brominating agent followed where necessary by removal of any protecting groups present;

with salt formation and conversion to a pharmaceutically acceptable derivative of the product of the above reaction as optional subsequent steps.

2. A process according to Claim 1 wherein the brominating agent is an N-bromo amide or a N-bromo imide.
3. A process according to Claim 1 or Claim 2 wherein the bromination is effected in the presence of a base.
4. A process according to Claim 3 wherein the base is an alkali metal bicarbonate.
5. A process according to any preceding claim wherein the brominating agent is N-bromosuccinimide and the bromination is effected in N,N-dimethylformamide solvent.
6. The 1R isomers of the compounds of formulae (II), (III), (IV) and (V).
7. The 1S isomers of the compounds of formula (VI), (VII) and (VIII).
8. Use of a compound according to Claim 1 and physiologically acceptable salts and solvates and pharmaceutically acceptable derivatives thereof for the manufacture of a medicament for the treatment or prophylaxis of Herpetoviridae infections in a human subject.

Claims for the following Contracting State: ES

1. A process for the preparation of [1R-(1β,3α,4β)]-5-[(E)-2-bromovinyl]-1-[3-hydroxy-4- (hydroxymethyl)-1-cyclopentyl-2,4-(1H,3H)-pyrimidinedione and salts, solvates and pharmaceutically acceptable derivatives thereof which comprises treating the 1R isomer of a compound of formula (II)

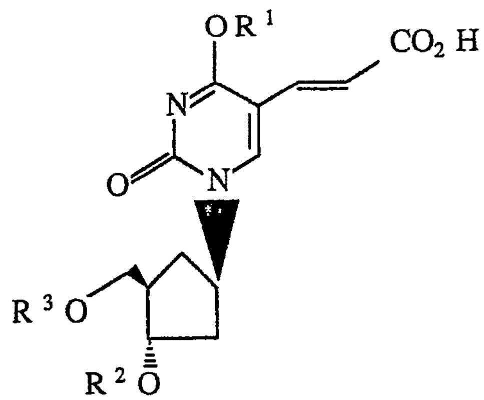

(II)

or a salt thereof (where $R^1$, $R^2$ and $R^3$, which may be the same or different, represent hydrogen atoms or protecting groups) with a brominating agent followed where necessary by removal of any protecting groups present;
with salt formation and conversion to a pharmaceutically acceptable derivative of the product of the above reaction as optional subsequent steps.
2. A process according to Claim 1 wherein the brominating agent is an N-bromo amide or a N-bromo imide.
3. A process according to Claim 1 or Claim 2 wherein the bromination is effected in the presence of a base.
4. A process according to Claim 3 wherein the base is an alkali metal bicarbonate.
5. A process according to any preceding claim wherein the brominating agent is N-bromosuccinimide and the bromination is effected in N,N-dimethylformamide solvent.
6. Use of a compound according to Claim 1 and physiologically acceptable salts and solvates and pharmaceutically acceptable derivatives thereof for the manufacture of a medicament for the treatment or prophylaxis of Herpetoviridae infections in a human subject.